# EUROPEAN PATENT APPLICATION

(11) **EP 1 908 745 A1**
(43) Date of publication of application: **09.04.2008**
(21) Application number: 06121919.2
(22) Date of filing: 06.10.2006
(51) Int. Cl.: C07C 17/275, C07C 19/10

(54) **Process for the preparation of halogenated hydrocarbons with at least 3 carbon atoms by reacting CFCl3 with an olefin**

(71) Applicant: SOLVAY (Société Anonyme), 1050 Bruxelles (BE)
(72) Inventor: Mathieu, Véronique, 1300 Wavre (BE); Mross, Stefan, 1030 Brussels (BE)
(74) Representative: Jacques, Philippe

(57) **Abstract**

A telomerisation process is described whereby fluorotrichloromethane is added to olefins like 2-chloroprop-1-ene, preferably in the presence of a catalyst. The reaction products, e.g. 1,1,3,3-tetrachloro-1-fluorobutane, can be further fluorinated.

## Description

The instant invention concerns a process for the preparation of halogenated hydrocarbons comprising at least 3 carbon atoms by catalytic reaction between a haloalkane and an olefin.

Halogenated hydrocarbons can be prepared by the addition of haloalkanes to olefins.

EP-A-0 787707 (US-A 5917098) discloses the preparation of 1,1,1,3,3-pentachlorobutane from tetrachloromethane and 2-chloroprop-1-ene in the presence of a copper halide and certain amines.

WO 98/50330 (US-A 6399839) discloses the preparation of chloroalkanes or chlorofluoroalkanes by the addition of haloalkanes, such as tetrachloromethane, 1,1,1-trichloroethane or 1,1,1-trichloro-2,2,2-trifluoroethane to olefins which may contain halogen atoms. In that process, an organically substituted copper compound is used as catalyst; a polar solvent and/or a cocatalyst selected among amines, amides and trialkyl phosphinoxides. Especially preferred compounds to be prepared are 1,1,1,3,3-pentachlorobutane and 1,1,1,3,3-pentachloropropane.

WO 98/50329 (US-A 6399840) discloses the preparation of 1,1,1,3,3-pentachlorobutane from tetrachloromethane and 2-chloroprop-1-ene in the presence of a copper (I) or copper (II) catalyst.

WO 97/07083 (US-A 5902914) discloses a process for the preparation of halogenated hydrocarbons by the addition of 0alkanes to olefins in the presence of copper chloride and t-butylamine as cocatalyst.

Object of the present invention is to provide a novel process for the preparation of halogenated hydrocarbons.

This object and other objects are achieved by the process of the present invention.

The present invention concerns a process for the preparation of halogenated hydrocarbons with at least 3 carbon atoms by the reaction of CFCl₃ and an olefin. Preferably, the addition reaction is performed in the presence of a catalyst.

The olefin which is used as starting material in the process of the present invention is generally ethylene, propylene or a butene, each of which may be substituted by one ore more halogen atoms, alkyl groups, halogenoalkyl groups, nitrile (CN) groups or carboxylic acid groups (COOH). Halogenated olefins are preferred. Chlorinated olefins are very suitable. Generally, they correspond to the formula R¹R²C=CClCR³. In this formula, R¹, R² and R³ independently represent H or C1, linear, branched or cyclic alkyl or alkenyl, an aryl or a heteroaryl group. These alkyl, alkenyl, aryl or heteroaryl groups may be substituted. Examples for such halogenated olefins are vinyl chloride, vinylidene chloride, trichloroethylene, the isomers of chloropropene like 1-chloroprop-1-ene, 2-chloroprop-1-ene, and 3-chloroprop-1-ene. 2-chloroprop-1-ene is especially preferred.

The halogenated hydrocarbons obtained by the process of the present invention preferably belong to the family of chlorofluoropropanes, chlorofluorobutanes and chlorofluoropentanes. The carbon atoms of the chlorofluoropropanes, chlorofluorobutanes and chlorofluoropentanes can be substituted by other functional groups like other halogen atoms (e.g. bromine or iodine), alkyl groups, halogenoalkyl groups, nitrile (CN) groups or carboxylic acid groups (COOH). Chlorofluoropropanes, chlorofluorobutanes and chlorofluoropentanes not substituted by such other functional groups are preferred.

Halogenated hydrocarbons of the general formula CₙH₍₂ₙ₊₂₎₋ₚ₋₁ClₚF are especially preferred reaction products. In this formula, n is an integer and stands for 3 or 4, p is an integer and stands for 2,3, 4, 5 or 6. Examples of compounds which can be produced by the process of the present invention are 1,1,3,3-tetrachloro-1-fluoropropane, 1,1,3,3-tetrachloro-1-fluorobutane, 1,1,3-trichloro-1-fluoropropane, 1,1,3-trichloro-1-fluorobutane and 1,1,3,3,3-pentachloro-1-fluoropropane. 1,1,3,3-tetrachloro-1-fluorobutane is preferred.

The catalyst can be selected from those catalysts which have been found suitable for telomerisation reactions concerning chloroalkanes and olefins. Optionally, cocatalysts can be present. The cocatalysts can also be selected from those catalysts which have been found suitable for said telomerisation reactions.

Very suitable are catalysts based on Cu salts. WO 98/50329 discloses certain Cu (I) and Cu (II) salts also suitable as catalyst for the present invention. Inorganic salts, like the halides, especially the chlorides and iodides, and organic salts are suitable. The organocopper compound suitable as catalyst in the process according to the present invention may be a compound formed with an organic acid compound. Carboxylic acids such as formic acid, acetic acid, propionic acid, butyric acid, acetylacetic acid, cyclo-hexanebutyric acid and benzoic acid typically constitute organic acid compounds. Chloro- or fluorocarboxylic acids, such as trichloroacetic acid and trifluoroacetic acid, are also suitable. Sulphonic acids, sulphinic acids and phosphonic acids, as well as chloro or fluoro derivatives thereof, may also be suitable. Other organic acid compounds are compounds having a hydrogen atom close to one or more electron-withdrawing groups such as carbonyl (C-O), nitrile (CN), sulphone (SO₂R), nitro (NO₂) and phenyl groups, as well as chloro or fluoro derivatives thereof. Among the organic acid compounds according to this definition, mention may be made in particular of acetylacetone, trifluoroacetylacetone, 1,1,1,5,5,5-hexafluoropentane-2, 4-dione, acetonitrile, ethyl acetoacetate, nitromethane, diphenylmethane, phenol and dimethyl sulphone. Copper compounds formed with organic acid compounds such as those mentioned above can be used in the process according to the present invention. The copper compounds formed with compounds such as acetylacetone, ethyl acetoacetate, acetic acid or cyclohexanebutyric acid and the chloro and fluoro derivatives thereof, are preferred. Copper (II) compounds are particularly preferred. Advantageously, the catalyst in the process according to the present invention is chosen from copper (II) acetate, copper (II) cyclohexanebutyrate and copper (II) acetylacetonate. Among organosubstituted copper compounds, preference is most particularly given to the compound formed between copper (II) and acetylacetone (copper (II) acetylacetonate, abbreviated as Cu(acac)₂). Iron, iron chloride or copper cyanide are also suitable as catalysts. A very preferred catalyst is Cu (II) chloride.

In a first embodiment of the process according to the invention, the reaction is carried out in the presence of a solvent. Any solvent in which the reagents form the desired product in satisfactory yield can be used. Advantageously, the reaction solvent is an alcohol, a nitrile, an amide, a lactone, a trialkylphosphine oxide or another polar solvent. Oxygen-containing solvents (i.e. solvents in which the molecule contains at least one oxygen atom) are preferred.

Among the alcohols which can be used as reaction solvent are, in particular, methanol, ethanol, isopropanol and tert-butanol. Among the nitriles which can be used as reaction solvent are, in particular, aliphatic nitriles, in particular acetonitrile, propionitrile or adiponitrile, and aromatic nitriles, in particular benzonitrile or tolunitrile. Among the nitriles, propionitrile and adiponitrile are preferred. Among the amides which can be used as reaction solvent are linear amides such as N,N-dimethylacetamide and N,N-dimethylformamide, and cyclic amides such as N-methylpyrrolidone. Mention may also be made of hexa-methylphosphoramide. Among the lactones which can be used as reaction solvent, mention may be made in particular of [gamma]-butyrolactone. Among the trialkylphosphine oxides which can be used as reaction solvent, mention may be made in particular of the compounds of formula (R⁴R⁵R⁶)PO, in which R⁴, R⁵ and R⁶ represent identical or different, preferably linear C3-C10 alkyl groups. Tri (n-butyl)-phosphine oxide, tri(n-hexyl)phosphine oxide, tri(n-octyl)phosphine oxide, n-octyldi(n-hexyl)phosphine oxide and n-hexyldi(n-octyl)phosphine oxide and mixtures thereof are selected in particular. As other polar solvents, mention may also be made of 1,3-dimethyl-2-imidazolidinone, dimethyl sulphoxide and tetrahydrofuran. Preferably, the solvent is an amide or a trialkylphosphine oxide. Good results have been obtained in particular with N-methylpyrrolidone, with N,N-dimethylacetamide and with a mixture of tri (n-hexyl)phosphine oxide, tri(n-octyl)phosphine oxide, n-octyldi(n-hexyl)phosphine oxide and n-hexyldi(n-octyl)-phosphine oxide.

The amount of solvent is not critical. A solution which is too diluted may not be favorable in view of efficiency and of the conversion to be achieved. Generally, the molar ratio between solvent and olefin is equal to or higher than 0.05, advantageously, equal to or higher than 0.1. Generally, the molar ratio between solvent and olefin is equal to or lower than 20, advantageously, equal to or lower than 15. Preferably, this ratio is equal to or higher than 0.2 and equal to or lower than 10. In the reaction medium, the molar ratio between solvent and catalyst can vary between 5 and 500, preferably 10 to 200.

In another embodiment, the reaction can be performed in the presence of a cocatalyst. Suitable cocatalysts are for example, amines, amides and oxides of trialkylphosphines.

Among the amines, aliphatic amines are preferred. Among primary, secondary and tertiary amines, primary amines are especially preferred. For example, n-butyl amine, t-butylamine, n-propylamine, isopropylamine or benzylamine are very suitable. Among the amides, N-methylpyrrolidone and N,N-dimethylformamide are to be mentioned. Among the trialkylphosphine oxides which can be used, mention may be made in particular of the compounds of formula (R⁴R⁵R⁶)PO, in which R⁴, R⁵ and R⁶ represent identical or different, preferably linear C3-C10 alkyl groups. Tri (n-butyl)-phosphine oxide, tri(n-hexyl)phosphine oxide, tri(n-octyl)phosphine oxide, n-octyldi(n-hexyl)phosphine oxide and n-hexyldi(n-octyl)phosphine oxide and mixtures thereof are selected in particular. A suitable mixture comprises tri (n-hexyl)phosphine oxide, tri (n-octyl)phosphine oxide, n-octyldi(n-hexyl)phosphine oxide and n-hexyldi(n-octyl)-phosphine oxide.

Other suitable cocatalysts are N-heterocyclic compounds. Preferred compounds of this type are disclosed in WO 97/05089 and WO 97/05090. The catalysts disclosed there belong to the group consisting of imidazoles, imidazolines, oxadiazoles, oxazoles, oxazolines, isoxazoles, thiazoles, thiazolines, pyrrolines, pyridines, trihydropyrimidines, pyrazoles, triazoles, triazolium salts, isothiazoles,tetrazoles, tetrazolium salts, thiadiazoles, pyridazines, pyrazines, oxazines and dihydrooxazine. 2-Alkyloxazolines, especially 2-alkyloxazolines, wherein alkyl denotes C1 to C4 alkyl, especially ethyl, are suitable.

If desired, in a discontinuous process, the cocatalyst can be added progressively, and is at least partially recovered for reuse. Such an embodiment is disclosed in WO 01/25175.

The recovery of the cocatalyst can be performed as described in WO 01/25177 from an aqueous phase by adding at least one base to it and recovering the cocatalyst from the aqueous phase.

In a discontinuous process, the molar ratio between catalyst and olefin often is greater than or equal to 0.001. Advantageously, it is equal to or greater than 0.002. Preferably, it is equal to or greater than 0.005. Often, the molar ratio between catalyst and olefin is lower than or equal to 5. Advantageously, it is lower than or equal to 1. Preferably, it is lower than or equal to 0.5.

In a continuous process, the molar ratio between catalyst and the olefin should lie in the range given above for a discontinuous process, but it may reach higher upper limits, e.g. it could be up to 10; here, the upper limit is preferably lower than or equal to 1.

In the second embodiment of the process according to the invention, the molar ratio between the cocatalyst and the olefin is generally greater than or equal to 0.01. Preferably, this molar ratio is greater than or equal to 0.05. Advantageously, this molar ratio is greater than or equal to 0.1. However, this molar ratio is usually less than or equal to 2. Preferably, this molar ratio is less than or equal to 1. Advantageously, this molar ratio is less than or equal to 0.5. The amount of cocatalyst used can vary, on a molar basis, from about 0.1 to about 200 times the amount of catalyst, preferably from about 0.5 to about 100 times.

The amount of catalyst and cocatalyst is expressed in a discontinuous process relative to the initial concentration of the olefin. In a continuous process, it is relative to the stationary concentration of the olefin in the reactor.

If desired, a solvent can be present during the reaction. Preferred solvents are aprotic. The trichlorofluoromethane may be used in excess and function as a reactant and as a solvent. If desired, the reaction products may be used as a solvent. Toluol is another suitable solvent, as well as hydrofluorocarbons like 1,1,1,3,3-pentafluorobutane.

The molar ratio between trichlorofluoromethane and the olefin, e.g. 2-chloroprop-1-ene, can vary in a broad range. In general, the ratio is equal to or greater than 0.1. Advantageously, it is equal to or greater than 0.5. Preferably, it is equal to or greater than 1. Generally, the ratio is equal to or lower than 20. Advantageously, it is equal to or lower than 10. Preferably, it equal to or lower than 8. In case of such high ratios, depending on the pressure, the trichlorofluoromethane also serves as a solvent.

Generally, the reaction is performed above ambient temperature. Preferably, the temperature is equal to or higher than 80°C. Advantageously, the reaction temperature is higher than or equal to 100°C. Generally, the temperature is equal to or lower than 160°C. Advantageously, it is lower than or equal to 130°C.

The reaction time in a discontinuous process or the residence time in a continuous process is dependent from parameters such as reaction temperature, catalyst concentration, concentration of the starting materials and the molar ratio of the components in the reaction mixture. Generally, the reaction time or residence time can vary from 5 seconds to 20 hours.

The pressure in the reactor is usually equal to or greater than ambient pressure. It is usually lower than or equal to 15 bars (abs.), preferably lower than or equal to 10 bars.

The process according to the present invention allows for the preparation of halogenated alkanes in an efficient manner. The alkanes produced are especially suitable as intermediates in chemical synthesis. For example, they can be fluorinated. The fluorinated products are useful, for example, as solvents, refrigerants or blowing agents. A part or, preferably, all of the chlorine atoms are substituted by fluorine atoms. The fluorination can be easily accomplished by reacting the halogenoalkanes obtained by the process of the present invention with HF which advantageously is anhydrous. The chlorine-fluorine exchange can be performed with or without added fluorination catalyst. Suitable fluorination catalysts are salts of antimony, salts of titanium, salts of tantalum or salts of tin. The halide salts, especially the fluorides, chlorides or chlorofluorides are preferred salts. Other suitable fluorination catalysts are compounds of chromium, aluminium and zirconium; the oxides are preferred compounds for catalyzing fluorination reactions.

Specific examples of hydrofluorocarbons produced by fluorination have the general formula CₙH₍₂ₙ₊₂₎₋ₚFₚ. In this formula, n is an integer and is 3 or 4, and p is an integer and is 3, 4, 5, 6 or 7. Especially preferred hydrofluorocarbons are 1,1,1,3,3-pentafluoropropane, 1,1,1,3,3,3-hexafluoropropane and 1,1,1,3,3-pentafluorobutane.

The following example is intended to explain the invention further without limiting it.

### Example 1:

2-chloroprop-1-ene, tetrachloromethane, CuC12 and t-butylamine were mixed in a stainless steel autoclave; the molar ratio between the olefin, the haloalkane, the copper compound and the amine was 1:2:0.0021:0.112. The mixture was heated up to 120 °C. At the outcome of the reaction (after 16 h), conversion of 2-chloroprop-1-ene was 84.5 %, and selectivity to 1,1,3,3-tetrachloro-1-fluorobutane was 51 %.

## Claims

1. Process for the preparation of halogenated hydrocarbons with at least 3 carbon atoms by the reaction of a CFCl₃ and an olefin, preferably in the presence of a catalyst.

2. Process according claim 1 wherein the reaction is performed in the presence of a copper (II) catalyst.

3. Process according to claim 1 or 2 wherein the olefin corresponds to the formula R¹R²=CClCR³, wherein R¹, R² and R³ independently represent H or Cl, linear, branched or cyclic alkyl or alkenyl, an aryl or a heteroaryl group, whereby the alkyl, alkenyl, aryl or heteroaryl groups may be substituted.

4. Process according to claim 3 wherein the olefin is selected from the group consisting of vinyl chloride, vinylidene chloride, trichloroethylene, and the isomers of chloropropene like 1-chloroprop-1-ene, 2-chloroprop-1-ene, and 3-chloroprop-1-ene.

5. Process according to claim 1 wherein the reaction is performed in the presence of a cocatalyst.

6. Process according to claim 5 wherein the cocatalyst is selected from the group consisting of amines, amides and trialkylphosphinoxides.

7. Process according to claim 1 wherein the reaction is performed in the presence of a solvent.

8. Process according to claim 7 wherein the solvent is selected from the group consisting of alcohols,nitriles, amides, lactones, and trialkylphosphine oxides.

9. Process for the preparation of hydrofluorocarbons wherein a halogenated hydrocarbon prepared according to any one of claims 1 to 8 is fluorinated.
